# EUROPEAN PATENT SPECIFICATION

(11) **EP 2 387 065 B1**
(45) Date of publication and mention of the grant of the patent: **26.08.2015**
(21) Application number: 11250524.3
(22) Date of filing: 12.05.2011
(51) Int. Cl.: H01J 61/12, H01J 61/82, A61N 5/06

(54) **A high pressure discharge lamp for collagen regeneration in the skin**
Hochdruckentladungslampe zur Kollagenregenerierung der Haut
Lampe à décharge haute pression pour la régénération de collagène de la peau

(30) Priority: 13.05.2010 IN DL11282010
(43) Date of publication of application: 16.11.2011
(73) Proprietor: Flowil International Lighting (Holding) B.V., 1097 JB Amsterdam (NL)
(72) Inventor: Hooker, James, Isle of Skye, IV45 8RU (GB); Derhaeg, Lode, 3290 Diest (BE)
(74) Representative: Hilger, Jens

(56) References cited:
- EP-A1- 1 156 512
- EP-A1- 1 288 998
- WO-A2-2006/106464
- WO-A2-2007/029151
- GB-A- 1 370 020
- US-A- 3 753 019
- US-A- 4 012 655
- US-A1- 2008 215 124
- US-B1- 6 847 167

## Description

### TECHNICAL FIELD:

The present disclosure relates in general, to a high pressure discharge lamp and more particularly to a high pressure discharge lamp for collagen regeneration in the skin to reduce the formation of wrinkles and present a youthful appearance.

### DESCRIPTION OF THE RELATED ART:

It is emerging from scientific studies in the literature that the suppression of wrinkles in the skin, so as to present a more youthful appearance, can be stimulated by irradiation with very high energy levels of light having a particular spectral power distribution. In particular, the benefits of red light have been cited, these long wavelengths stimulating the formation of collagen as well as fibroblasts - both of which increase the elasticity of the skin and lead to a tightening effect of the surface which has been shown to have a positive effect in terms of removing wrinkles. A concise overview of the process is referenced, for instance, in the book Hautalterung by Jean Krutmann and Thomas Diepge, published 2008 by Springer Verlag - in particular Chapter 8 from pp.113-132. The conventional techniques for anti-wrinkle treatment are following.
1. Skin ablation technique- It is a slow and painful technique. It involves physical destruction or removal of the upper layers of skin such that new skin will be produced as part of the natural wound healing process. Some new collagen is produced in parallel with the new skin generation.
2. Cosmetic creams- The effect is limited, expensive and very dependent on application quality.
3. Lasers- They are suitable only for treatment of small areas of the skin at a time; with light of a frequency which either directly stimulates the production of new collagen, or causes burning and destruction of the skin, which will subsequently heal and generate new collagen in the process. This technique is expensive. The safety implications of working with laser radiation restricts use of this equipment to hospitals by skilled persons who have received substantial training, and this prevents such equipment from being further commercialised to serve the wider commercial and residential demand for collagen stimulation devices.
4. LEDs- Owing to the low total output power of LEDs, large arrays comprising many hundreds or even thousands of LEDs are necessary to achieve the required irradiation levels. The LEDs must often be mounted at inconveniently short distances from the skin, sometimes in direct contact with the skin. The clustering of many LEDs into a small area presents difficult thermal challenges for the irradiation unit in terms of the extraction of the heat produced. Additionally, the LEDs radiate only a very narrow bandwidth spectrum, and often it is desirable that skin rejuvenation is executed using various different wavelengths of light applied simultaneously. This can only be achieved by employing multiple different types of LEDs each having distinct spectral power distributions.
5. Low pressure discharge lamps- These have large physical dimensions, and although they are efficient and can be produced at low costs, they cannot easily achieve the high irradiation levels that are desired for optimal collagen regeneration in the skin.

Publication WO 2004/075985 provides elaborate details concerning the various photochemical mechanisms, which drive the referenced skin rejuvenation processes, and claims a method of treatment involving the use of certain narrow bandwidth wavelength ranges of light. Said narrow bandwidth wavelengths of light are generated by non-laser light, moreover by employing various standard solid-state (LED) light sources already on the market and which generate the wavelength ranges of interest. The LEDs employed may all be of the same type so as to produce light of only one narrow bandwidth wavelength range, but the virtues of combining several different LED types together so as to realise a polychromatic light source consisting of a plurality of narrow bandwidth wavelength ranges are extolled.

Although the existing solid-state LED light sources are capable of generating the narrow bandwidth wavelength ranges disclosed, they are not particularly efficient light sources and are crippled by the aformentioned caveats of low output power and extremely high cost.

Publication no WO 2007/029151A2 relates to a UV lamp for treatment of the skin. The lamp has a radiation capacity substantially in the UV spectrum and in the red-light spectrum. It combines UV and collagen light. It is well known that exposure of the skin to ultra-violet radiation for instance during artificial sun tanning, leads to destruction of collagen and accelerates the formation of wrinkles. The publication WO 2007/029151A2 teaches the construction of an ultra-violet lamp whose spectrum also comprises a proportion of red light in the region 600-700nm, so as to partially offset the damaging effects of the ultra-violet radiation by the beneficial effects of the red light. Further, the metal halide chemistry claimed in WO 2007/029151A2 are Lithium and/or Calcium iodides, which radiate primarily in the region 630-700nm.

US publication no. 2008/0215124 relates to an apparatus for cosmetic skin rejuvenation treatment comprising a continuous wave light source accommodating a high-intensity discharge lamp, the high-intensity discharge lamp having at least one predominant spectral peak between 550 and 700 nm, preferably between 650 and 700 nm. Thus it provides an apparatus which can receive any standard lamp or be changed for a similar coloured metal halide lamp having a substantially blue coloured spectrum, or another having a substantially red coloured spectrum. The choice of lamp type can be changed to treat different medical conditions. The lamp which is referenced for collagen treatment describes standard coloured lighting lamps which are available for coloured floodlighting effects and comprise at least a Lithium iodide filling and produce a spectral peak in the wavelength range mentioned, and could be installed without modification in the depicted apparatus to perform the functions described.

US publication no 2002/0195943 relates to lamps, wherein the light source includes light emitting plasma contained within an arc tube having dichroic thin film coatings to improve the operating characteristics of the lamp. The high intensity discharge lamp having a vaparisable fill material comprises halides of sodium, scandium, and thorium. The coating forms a notch filter that reflects nearly the entire incident light in a narrow band substantially centered on a wavelength of about 590 nm, and transmits nearly eighty percent of the incident light in the visible spectrum and outside of that narrow band. These types of lamps are ineffective light sources for collagen stimulation in the skin, because the coating is designed so as to filter out wavelengths around 590nm. Moreover, if the teachings of US 2002/0195943 were modified so as to allow transmission of the wavelengths from 590-650nm which are desirable for collagen treatment, while blocking the transmission of the other wavelengths, said lamp would still be rather inefficient as a collagen light source because energy is first being wasted in generating light outside the range 590-650nm, said radiation then being filtered out by the claimed coating.

US publication no 2003/0141818 relates to a metal halide discharge lamp for the generation of visible radiation. Ihe metal halide lamp comprises an arc tube formed of a material transmissive to visible radiation and has superior red colour rendering characteristics A glass shroud enclosing the arc tube is used as a narrow band filter to reduce radiation at about 585 nm with a half peak bandwidth of between about 5 and 40 nm and the lamp fill comprises mercury plus either Ar or Xe plus halides of at least one of the elements of Dy, Ho, Tm, Na, Li, Cs. This is a ceramic metal halide lamp having increased red colour rendering index (the R9 value), originating from calcium whose vapour pressure is boosted by chemical complexing with Aluminium or Gallium, and further enhanced by addition of Thallium whose strong self-absorption filters out part of the spectrum, and still further enhanced by use of a neodymium doped quartz shroud around the arc tube to filter out undesirable yellow light. US publication no 2007/0085482 also relates to high intensity arc discharge metal halide lamps having improved red color rendition while retaining high efficacy. If the lamp described in this publication was applied to collagen treatment of the skin it would be rather inefficient, because light is first generated of all wavelengths, the undesired wavelengths then being filtered out which represents a waste of energy.

Further reference is made to European patent application EP 1 156 512 A which discloses a high-pressure discharge lamp for photodynamic therapy, producing light in the range from 600 to 800 nm.

The collagen treatment effect has been reported and confirmed in numerous studies in which Laser and/or LED light sources have been employed to treat small areas of the skin- There is a consequent desire to commercialise the technology for facial and/or full body treatment, however the cost and safety aspects of working with laser sources makes this difficult. Meanwhile, the total output power of LEDs is quite low and they only radiate light of one particular colour or narrow-bandwidth range of wavelengths, and are mainly useful only when mounted at inconveniently short distances from the skin. They also bring exceptionally high cost implications. Commercial systems are thus entering the market based on low pressure tubular fluorescent discharge lamps which are enjoying considerable success However the power density that can be achieved from these large volume light sources is not really sufficient to achieve the desired levels of collagen regeneration - especially for facial treatment. A consequence of the low irradiance levels is that the necessary duration of the treatment is inconveniently long. To reduce the treatment time to acceptable levels, a pbotosensitisor cream or drug is typically applied before treatment either exogenously or endogenously, which serves to increase the sensitivity of the skin to incident light. This process is particularly inconvenient and may lead to potentially undesirable side-effects, and furthermore its effects remain for many hours after cessation of the photo-rejuvenation treatment. Thus persons venturing into the outdoor sunlight following phototherapy are undesirably susceptible to sunburn until sufficient time has elapsed that the photosensitivity of their skin relaxes to normal levels.

Thus there is a need to realize a new light source which delivers the required light spectrum for optimal skin penetration and collagen regeneration from a compact and high intensity light source, which realizes high efficiency in the production of the collagen active radiation and can be manufactured and operated at low costs comparable to other light sources commonly employed in body care applications, and preferably having a sufficiently high intensity that it can stimulate the collagen regeneration process on its own without reliance on photosensitizing creams or other drugs.

### OBJECTS:

The object of the present disclosure is to provide a high pressure discharge lamp for collagen regeneration in the skin to reduce the formation of wrinkles and present a youthful appearance.

Another object of the present disclosure is to provide a high pressure discharge lamp which obtains very high irradiance levels for the regeneration of the collagen in the skin.

Yet another object of the present disclosure is to produce the optimal spectral power distribution of light for regeneration of collagen in the skin by irradiation, with as little energy as possible being radiated at other wavelengths that are less desirable for the intended photo-rejuvenation applications.

Still another object of the present disclosure is to provide a high pressure discharge lamp for collagen regeneration in the skin to reduce the formation of wrinkles and present a youthful appearance without any harmful effects in terms of irradiation on the human body i.e. by reducing presence of potentially damaging ultra-violet wavelengths to the lowest extent possible.

Further object of the present disclosure is to provide a high pressure discharge lamp for collagen regeneration in the skin to reduce the formation of wrinkles and present a youthful appearance which does not rely on any physical contact with the skin, or use of chemical products or require destruction or removal of upper skin layers to achieve the desired collagen regeneration effect.

A final object of the present disclosure is to optimise the spectral power distribution from the lamp so as to realise the maximum generation efficiency of collagen active radiation.

### SUMMARY:

In order to overcome the above mentioned problems and to achieve the said objects, the present disclosure provides a high pressure discharge lamp for collagen regeneration in the skin to reduce the formation of wrinkles and present a youthful appearance. The lamp produces the optimal spectral power distribution of light for regeneration of collagen and a subsequent increase in the elasticity of the skin by irradiation and obtains very high irradiance levels causing collagen regeneration without reliance on the exogenous or endogenous application of creams, drugs or other chemical products. The disclosure produces an increased proportion of light in the range of 590-650nm which is effective in the generation of new collagen fibers in the skin, so as to remove wrinkles and maintain a youthful appearance.

The high pressure discharge lamp for collagen regeneration in the skin comprises an arc tube equipped with tungsten electrodes characterised in that the arc tube is filled with a chemical mix comprising halides of sodium, lithium or any combination thereof, and the electrical loading is in the range 50 to 7 0 W/cm2 of arc tube surface area, so as to optimise the spectrum for realising the maximum generation efficiency of collagen active radiation.

The chemical mix in the arc tube comprises 0.1 to 5.0 wt% of indium iodide or indium bromide.

The use of indium iodide or bromide performs a role in correcting the colour point of the lamp.

The chemical mix optionally comprises 0.1% up to 30 wt% of the halides of zinc andior barium, calcium or strontium to increase the efficiency of generation of collagen active radiation.

The metal halide salts are dosed in both iodide and bromide form, wherein the ratio of hasides in iodide and bromide form is adjusted such that the bromine content does not exceed 30% of the total halogen dose weight so as to attain an optimum rate of blackening of the arc tube to ensure favourable maintenance of the generation of collagen active radiation as the lamp ages.

An arc shape controlling agent may be added, which has no substantial effect on the visible spectrum but may serve, to fatten the arc discharge so as to control the quartz arc tube wall temperature and hence the vapour pressure of the other elements. Such an agent may be caesium iodide up to 50 wt%.

The arc tube may be partially or completely coated with a thermally reflective material, for instance by applying a coating of alumina or zirconia in the vicinity of the electrodes at the ends of the arc tube. Said coating may extend either partially or completely aver the entire arc tube surface so as to further raise its operating temperature and hence raise the vapour pressures of the metal halide salts, thereby influencing the spectral power distribution in a beneficial fashion so as to improve the efficiency of generation of collagen active radiation.

The arc tube may be enclosed within a translucent shroud, for instance of quartz glass, which serves to increase its operating temperature and hence raise the vapour pressures of the metal halide salts, thereby influencing the spectral power distribution in a beneficial fashion so as to improve the efficiency of generation of collagen active radiation.

In an alternate embodiment, such as may be required for the construction of a single ended lamp in which the conventional double ended arc tube is mounted on a single base, it is customary for the electric current to be carried to the distal end of the arc tube by a metallic lead wire which is contiguously juxtaposed to and parallel to the arc tube. Presence of a metallic component adjacent to the are tube is known to cause loss of sodium from the arc tube due to photoemission of the metallic component. Therefore, in constructing a single ended version of the collagen IIID lamp it is desirable to circumvent said sodium-loss phenomenon by disposing the return lead within an opaque material, for instance a sleeve of ceramic. Alternatively the lamp may be equipped with an additional shroud of quartz glass, the arc tube being disposed within said shroud and the adjacent return lead wire being disposed entirely without said shroud and furthermore not in electrical contact with the shroud, such that the latter shall be free to acquire an electrically floating state.

The arc tube itself or a containing shroud may optionally be fabricated from a doped quartz containing additive oxides which serve to block the transmission of ultra-violet wavelengths, so as to achieve a reduced UV light source.

Said lamp can emit across the whole visible spectrum.

### BRIEF DESCRIPTION OF THE DRAWINGS:

It is to be noted, however, that the appended drawings illustrate only typical embodiments of this disclosure and are therefore not to be considered for limiting of its scope, for the disclosure may admit to other equally effective embodiments.
FIG. 1 shows a high pressure discharge lamp of the double ended variety, for collagen regeneration in skin treatment, in accordance with the present disclosure.
FIG. 2 shows a high pressure discharge lamp of the single ended variety, for collagen regeneration in skin treatment, in accordance with the present disclosure.
FIG. 3 shows a high pressure discharge lamp of the single ended variety, for collagen regeneration in skin treatment, in accordance with an alternate embodiment of the present disclosure.
FIG 4 shows a high pressure discharge lamp of the single ended variety, for collagen regeneration in skin treatment, in accordance with an alternate embodiment of the present disclosure.

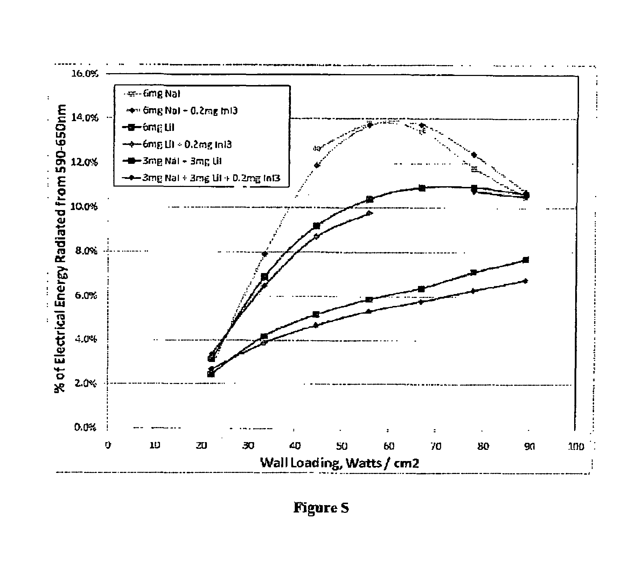

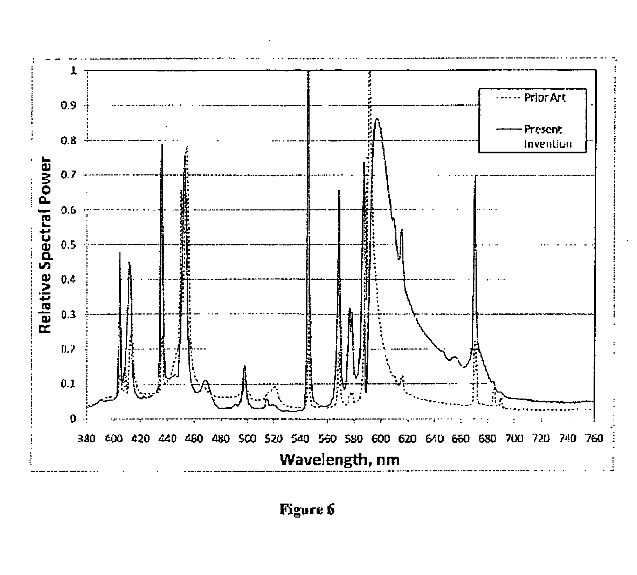

FIG. 5 is graph showing the influence of the wall loading on the efficiency of transforming electrical energy into collagen active radiation in the region 590-654nm, for various lamp chemistries. FIG. 6 shows in a dashed line the spectral power distribution of a typical coloured metal halide lamp according to the prior art, whose design is not optimised to achieve the highest proportion of its radiation in the region 590-650nm. The solid line represents a typical spectral power distribution produced by the lamp in the present application, in which it is clear that the proportion of radiation in the region 590-650nm has been tremendously enhanced.

### DESCRIPTION OF THE PREFERRED EMBODIMENTS:

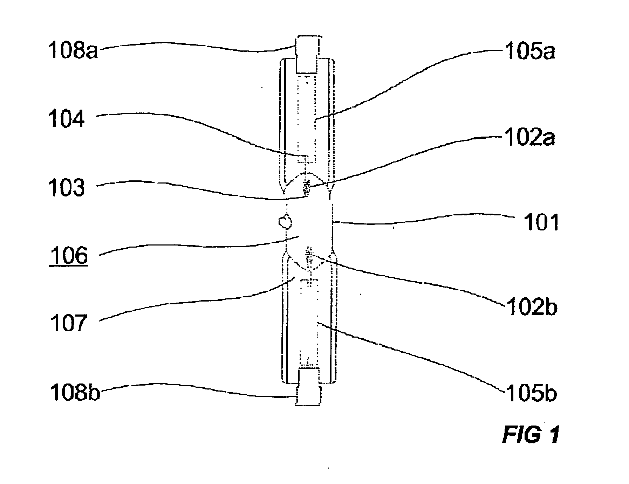

Reference may be made to Figure 1 which shows a high pressure discharge lamp of the double ended variety, for collagen regeneration in skin treatment. It comprises a quartz discharge tube (101) equipped with a pair of tungsten electrodes (102) whose distal ends (103) are projected into the interior of the discharge vessel and whose proximal ends (104) are welded to a short length of molybdenum sealing foil (105), these being hermetically sealed into the quartz containment by pinching of the quartz embodiment in its softened state around the electrode assemblies. The arc tube is characterised in that its interior volume (106) is filled with the metal halide chemistry claimed, plus an inert gas, for example argon or argon bearing a trace of radioactive krypton-85 to facilitate starting, and its dimensions are arranged such that the specified conditions of electrical loading are attained. Optionally a thermally-reflective coating (107) may be applied around the end chambers of the arc tube in the vicinity of the electrodes so as to eliminate the formation of a cold spot in this region. The lamp is equipped with two caps (108a, 108b) aligned on its axis of symmetry which effect an electro-mechanical interface with the equipment in which it is to be used.

The high pressure discharge lamp, according to the present disclosure, is a free-burning quartz metal halide lamp having an arc tube filled with a chemical mix under specified conditions of electrical wall loading. The spectral power distribution of the lamp has been optimised and matched to the skin transmission and collagen regeneration response curves by choosing the most suitable chemical dose in the arc tube which combines at least the halides of the elements Sodium and/or Lithium with Indium iodide or Indium bromide in the chemical dose.

The lamp is filled with a chemistry which comprises at least the iodides and/or bromides of Na and optionally also Li, as well as indium iodide or indium bromide. The elements Na and optionally Li generates the required red colour radiation. Indium iodide or indium bromide is employed to correct the colour point to a more intense red appearance. The admixture of the halides of Zinc and/or Barium, Calcimn or Strontium is beneficial in certain embodiments so as to further increase the efficiency of generation of collagen active radiation.

The electrical wall loading of the light source has been chosen such that it offers much higher output compared to traditional low pressure lamps and furthermore with increased generation efficiency of collagen active radiation compared to prior art discharge lamps, thus allowing for high irradiance levels over a large area. The power density is not so high, however, that it would fall under the same restrictive practices that concern the use of laser radiation.

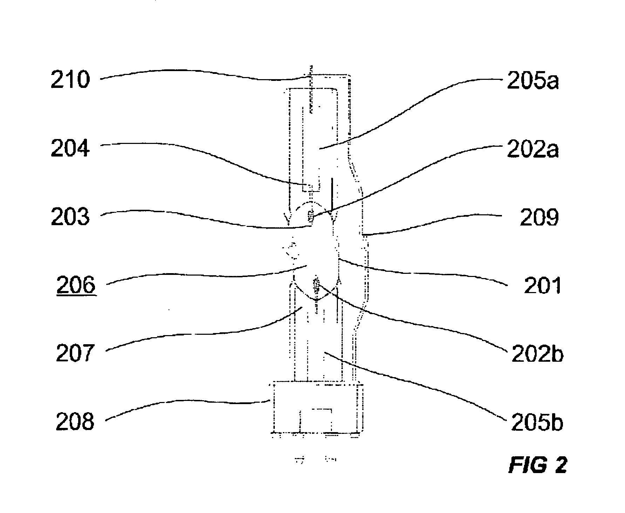

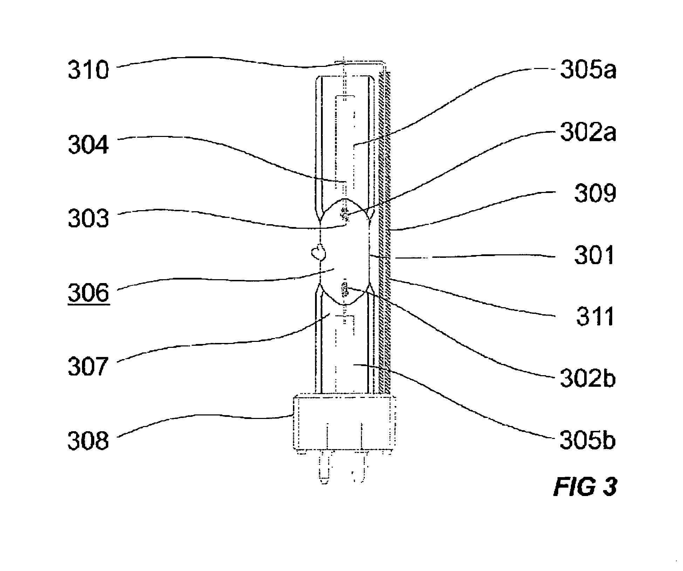

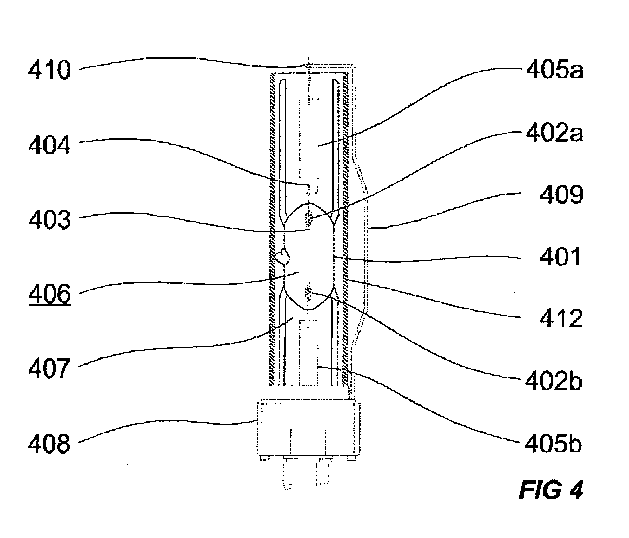

Figure 2 shows a high pressure discharge lamp of the single ended variety, for collagen regeneration in skin treatment. Its construction is substantially the same as the lamp of Figure 1 except that it is equipped with only a single cap (208), the electric current to the distal end (210) of the arc tube (201) being delivered by an outer return lead (209) which is contiguously juxtaposed to the axis of the arc tube and electrically connected to the cap (208) as well as the distal end of the arc tube. Figure 3 shows an improved high pressure discharge lamp of the single ended variety, for collagen regeneration in skin treatment- Its construction is substantially the same as the lamp of Figure 2 except that the return lead (309) is disposed substantially within an opaque ceramic tube (311) so as to shield the return lead (309) from high energy photons incident from the arc tube (301) and thereby interrupt the process of sodium loss from the arc tube. Figure 4 shows an improved high pressure discharge lamp of the single ended variety, for collagen regeneration in skin treatment. Its construction is substantially the same as the lamp of Figure 2 except that the arc tube (401) is disposed substantially within a quartz shroud (412), the adjacent return lead return lead (409) being disposed entirely without the quartz shroud (412) and furthermore not in electrical contact with the same, such that the quartz shroud (412) shall be free to acquire an electrically floating state and thereby interrupt the process of sodium loss from the arc tube. The thermal insulation role of the shroud (412) is also beneficial to the improvement of the lamp spectrum. Figure 5 is graph showing the influence of the wall loading on the efficiency of transforming electrical energy into collagen active radiation in the region 590-650nm, for various lamp chemistries, and indicating the general trend that efficiency is at a maximum when the wall loading is between about 50 to 70 W/cm2. Figure 6 shows in a dashed line the spectrum of a typical coloured metal halide lamp according to the prior art, whose design is not optimised to achieve the highest proportion of its radiation in the region 590-650nm. On the same axes is shown in a solid line the spectrum of a lamp made according to the present application, whose chemical filling and electrical Loading have been optimised so as to maximise its radiation in the region 590-650nm.

According to Figures 5 and 6, the disclosure produces an optimised spectral power distribution of light for regeneration of collagen in the skin by irradiation and obtains very high irradiance levels in the spectral area in the range of 590nm to 650nm causing collagen regeneration in the skin. The lamp produces intense red colour light which is effective in generation of new collagen fibers in the skin, and to remove wrinkles and maintain a youthful appearance. The spectral power distribution according to the disclosure has been optimised to the spectra sensitivity of the photochemical reactions which trigger the collagen regeneration process These small lamps with a high output of collagen active radiation can achieve the desired irradiation and dose levels in a short period of time. The resulting spectrum falls largely in an area having no known harmful effects in terms of irradiation on the human body. They do not rely on any physical contact with the skin, or use of chemical products, or require destruction or removal of upper skin layers to achieve the desired collagen regeneration effect. The present lamp is a low cost lamp compatible with existing high pressure light sources employed in body care applications.

The lamp according to the present disclosure has increased efficiency as it minimises the production of useless energy in the blue and green wavelength ranges, which are absorbed near to the surface of the skin.

It is to be noted that the present disclosure is susceptible to modifications, adaptations and changes by those skilled in the art. Such variant embodiments employing the concepts and features of this disclosure are intended to be within the scope of the present disclosure, which is further set forth under the following claims: -

## Claims

1. A high pressure discharge lamp for collagen regeneration in the skin comprising an arc tube equipped with tungsten electrodes **characterized in that** the arc tube is filled with a chemical mix comprising halides of sodium, lithium or any combination thereof and 0.1 to 5.0 wt% of indium iodide or indium bromide, and the electrical loading is in the range 50 to 70 W/cm2 of arc tube surface area, wherein the lamp is optimised to emit the maximum proportion of radiation in the range of 590 nm to 650 nm.

2. The high pressure discharge lamp as claimed in claim 1, wherein the chemical mix in the arc tube comprises a sodium halide.

3. The high pressure discharge lamp as claimed in claim 2, wherein the sodium halide is sodium iodide.

4. The high pressure discharge lamp as claimed in claim 1, wherein the chemical mix in the arc tube comprises a lithium halide.

5. The high pressure discharge lamp as claimed in claim 4, wherein the lithium halide is lithium iodide.

6. The high pressure discharge lamp as claimed in any of the preceding claims, wherein the chemical mix optionally comprises 0.1% to 30 wt% of the halides of zinc and/or barium, calcium or strontium to increase the efficiency of generation of collagen active radiation.

7. The high pressure discharge lamp as claimed in any of the preceding claims, wherein the metal halide salts are dosed in both iodide and bromide form.

8. The high pressure discharge lamp as claimed in claim 7, wherein the ratio of halides in iodide and bromide form is adjusted such that the bromine content does not exceed 30 % of the total halogen dose weight.

9. The high pressure discharge lamp as described in any of the preceding claims, wherein the arc tube is partially or completely coated with a thermally reflective material, preferably a coating of alumina or zirconia, in the vicinity of the electrodes at the ends of the arc tube.

## Patentansprüche

1. Hochdruckentladungslampe zur Kollagenregenerierung der Haut, die eine mit Wolframelektroden ausgestattete Bogenentladungsröhre umfasst, **dadurch gekennzeichnet, dass** die Bogenentladungsröhre mit einem Chemikaliengemisch gefüllt ist, das Halogenide von Natrium, Lithium oder eine beliebige Kombination davon und 0,1 bis 5,0 Gew.-% Indiumiodid oder Indiumbromid umfasst, und die elektrische Belastung im Bereich von 50 bis 70 W/cm² Bogenentladungsröhrenoberfläche liegt, wobei die Lampe zur Emission des maximalen Anteils an Strahlung im Bereich von 590 nm bis 650 nm optimiert ist.

2. Hochdruckentladungslampe nach Anspruch 1, wobei das Chemikaliengemisch in der Bogenentladungsröhre ein Natriumhalogenid umfasst.

3. Hochdruckentladungslampe nach Anspruch 2, wobei das Natriumhalogenid ein Natriumiodid ist.

4. Hochdruckentladungslampe nach Anspruch 1, wobei das Chemikaliengemisch in der Bogenentladungsröhre ein Lithiumhalogenid umfasst.

5. Hochdruckentladungslampe nach Anspruch 4, wobei das Lithiumhalogenid ein Lithiumiodid ist.

6. Hochdruckentladungslampe nach einem der vorhergehenden Ansprüche, wobei das Chemikaliengemisch nach Belieben 0,1 Gew.-% bis 30 Gew.-% der Halogenide von Zink und/oder Barium, Calcium oder Strontium umfasst, um die Effizienz der Erzeugung von kollagenaktiver Strahlung zu erhöhen.

7. Hochdruckentladungslampe nach einem der vorhergehenden Ansprüche, wobei die Metallhalogenidsalze in sowohl Iodid- als auch Bromidform dosiert sind.

8. Hochdruckentladungslampe nach Anspruch 7, wobei das Verhältnis von Halogeniden in Iodidform und in Bromidform derart eingestellt ist, dass der Bromgehalt nicht mehr als 30% des Halogendosisgesamtgewichts beträgt.

9. Hochdruckentladungslampe nach einem der vorhergehenden Ansprüche, wobei die Bogenentladungsröhre im Umfeld der Elektroden an den Enden der Bogenentladungsröhre teilweise oder völlig mit einem wärmereflektierenden Material beschichtet ist, vorzugsweise einer Beschichtung aus Aluminium- oder Zirkonoxid.

## Revendications

1. Lampe à décharge à haute pression pour la régénération du collagène dans la peau comprenant un tube à arc équipé d'électrodes de tungstène, **caractérisée en ce que** le tube à arc est rempli avec un mélange chimique comprenant des halogénures de sodium, lithium ou toute combinaison de ceux-ci et 0,1 à 5,0 % en poids d'iodure d'indium ou bromure d'indium, et la charge électrique se situe dans la gamme de 50 à 70 W/cm² de surface de tube à arc, la lampe étant optimisée pour émettre la proportion maximale de rayonnement dans la gamme de 590 nm à 650 nm.

2. Lampe à décharge à haute pression selon la revendication 1, dans laquelle le mélange chimique dans le tube à arc comprend un halogénure de sodium.

3. Lampe à décharge à haute pression selon la revendication 2, dans laquelle l'halogénure de sodium est l'iodure de sodium.

4. Lampe à décharge à haute pression selon la revendication 1, dans laquelle le mélange chimique dans le tube à arc comprend un halogénure de lithium.

5. Lampe à décharge à haute pression selon la revendication 4, dans laquelle l'halogénure de lithium est l'iodure de lithium.

6. Lampe à décharge à haute pression selon l'une quelconque des revendications précédentes, dans laquelle le mélange chimique comprend éventuellement 0,1 % à 30 % en poids des halogénures de zinc et/ou baryum, calcium ou strontium pour augmenter l'efficacité de production de rayonnement actif sur le collagène.

7. Lampe à décharge à haute pression selon l'une quelconque des revendications précédentes, dans laquelle les sels d'halogénures métalliques sont dosés à la fois sous forme d'iodure et de bromure.

8. Lampe à décharge à haute pression selon la revendication 7, dans laquelle la proportion d'halogénures sous forme d'iodure et de bromure est ajustée de telle sorte que la teneur en brome ne dépasse pas 30 % du poids de la dose totale d'halogènes.

9. Lampe à décharge à haute pression telle que décrite dans l'une quelconque des revendications précédentes, dans laquelle le tube à arc est partiellement ou complètement recouvert d'un matériau thermoréfléchissant, de préférence un revêtement d'alumine ou zircone, au voisinage des électrodes aux extrémités du tube à arc.
